# EUROPEAN PATENT SPECIFICATION

(11) **EP 2 061 536 B1**
(45) Date of publication and mention of the grant of the patent: **19.10.2011**
(21) Application number: 07841172.5
(22) Date of filing: 21.08.2007
(51) Int. Cl.: A61M 5/178, A61J 1/20, A61M 5/145, A61M 5/315

(54) **SYSTEMS AND METHODS ALLOWING FOR RESERVOIR FILLING AND INFUSION MEDIUM DELIVERY**
SYSTEME UND VERFAHREN ZUR FÜLLUNG VON BEHÄLTERN UND ZUR AUSGABE EINES INFUSIONSMEDIUMS
SYSTÈMES ET MÉTHODES PERMETTANT DE REMPLIR UN RÉSERVOIR ET DE DÉLIVRER UNE SUBSTANCE DE PERFUSION

(30) Priority: 23.08.2006 US 839821 P
(43) Date of publication of application: 27.05.2009
(73) Proprietor: Medtronic MiniMed, Inc., Northridge, CA 91325 (US)
(72) Inventor: MOUNCE, R. Paul, Burbank, California 91506 (US); MOBERG, Sheldon B., Thousand Oaks, California 91362 (US); HANSON, Ian B., Granada Hills, CA 91344 (US); BENTE, Paul, F. Iv, Los Angeles, California 90049 (US); KAVAZOV, Julian D., Arcadia, California 91006 (US); KOVELMAN, Paul H., Simi Valley, California 93063 (US); LUAN, Truong Gia, Winnetka, California 91306 (US)
(74) Representative: Ruschke, Hans Edvard
(86) International application number: PCT/US2007/076429
(87) International publication number: WO 2008/024781

(56) References cited:
- EP-A- 0 138 681
- EP-A- 0 614 653
- WO-A-01/68166
- WO-A-93/02723
- WO-A-98/17336
- WO-A-99/55401
- WO-A-03/008021
- FR-A- 2 783 433
- US-A- 2 957 609
- US-A- 3 999 543
- US-A- 4 191 225
- US-A- 4 932 937
- US-A- 5 232 029
- US-A1- 2006 178 641
- US-B1- 6 746 438
- US-B2- 6 591 876

## Description

### CROSS-REFERENCE TO RELATED PATENT APPLICATIONS

This application is related to published U.S. Patent Application US 2008/0097327 entitled "Systems and Methods Allowing for Reservoir Filling and Infusion Medium Delivery".

Embodiments of the present invention relate to published U.S. Patent Application US 2006/0264894.

Embodiments of the present invention relate to: (i) published U.S. Patent Applications US 2008/097381 and US 2008/051727 entitled "Infusion Medium Delivery Device and Method with Drive Device for Driving Plunger in Reservoir"; (ii) published U.S. Patent Application US 2008/0051698, entitled "Infusion Medium Delivery Device and Method with Compressible or Curved Reservoir or Conduit"; (iii) published U.S. Application US 2008/0051714 entitled "Infusion Medium Delivery System, Device and Method with Needle Inserter and Needle Inserter Device and Method"; and (iv) published U.S. Patent Application U.S. 2008/0097291 entitled "Infusion Pumps and Methods and Delivery Devices and Methods with Same."

Embodiments of the present invention also relate to: (i) published U.S. Patent Application US 2009/0270811 entitled "Infusion Medium Delivery Device and Method with Drive Device for Driving Plunger in Reservoir"; (ii) published U.S. Patent Application US 2008/0051698 entitled "Infusion Medium Delivery Device and Method with Compressible or Curved Reservoir or Conduit"; (iii) published U.S. Patent Application US 2009/0082728, entitled "Infusion Medium Delivery System, Device and Method with Needle Inserter and Needle Inserter Device and Method"; and (iv) published U.S. Patent Application US 2008/0097375 entitled "Infusion Pumps and Methods and Delivery Devices and Methods with Same".

### BACKGROUND OF THE INVENTION

1. Field of the Invention

Embodiments of the present invention relate generally to structures, systems, and methods allowing for reservoir filling and, in specific embodiments, to an infusion medium delivery system allowing for filling a reservoir with an infusion medium and for delivering the infusion medium to a patient.

2. Related Art

According to modem medical techniques, certain chronic diseases may be treated by delivering a medication or other substance to the body of a patient. For example, diabetes is a chronic disease that is commonly treated by delivering defined amounts of insulin to a patient at appropriate times. Traditionally, manually operated syringes and insulin pens have been employed for delivering insulin to a patient. More recently, modem systems have been designed to include programmable pumps for delivering controlled amounts of medication to a patient. However, some programmable pump delivery systems operate for only a prescribed period of time and require disposal when one or more system components have exceeded an operational lifetime, even if other system components are still operational.

Pump type delivery devices have been configured in external devices, which connect to a patient, and have also been configured in implantable devices, which are implanted inside of the body of a patient. External pump type delivery devices include devices designed for use in a stationary location, such as a hospital, a clinic, or the like, and further include devices configured for ambulatory or portable use, such as devices that are designed to be carried by a patient, or the like. External pump type delivery devices may be connected in fluid flow communication to a patient or user, for example, through a suitable hollow tubing. The hollow tubing may be connected to a hollow needle that is designed to pierce the skin of the patient and to deliver an infusion medium there-through. Alternatively, the hollow tubing may be connected directly to the patient as or through a cannula, or the like.

Examples of some external pump type delivery devices are described in the following references: (i) Published PCT Application WO 01/70307 (PCT/US01/09139), entitled "Exchangeable Electronic Cards for Infusion Devices"; (ii) Published PCT Application WO 04/030716 (PCT/US2003/028769), entitled "Components and Methods for Patient Infusion Device"; (iii) Published PCT Application WO 04/030717 (PCT/US2003/029019), entitled "Dispenser Components and Methods for Infusion Device"; (iv) U.S. Patent Application Pub. No. 2005/0065760, entitled "Method for Advising Patients Concerning Doses Of Insulin"; and (v) U.S. Patent No. 6,589,229, entitled "Wearable Self-Contained Drug Infusion Device".
An example of a syringe, which is attachable to a powered drive member of an injector over a range of relative axial positions of the syringe plunger and the drive member, is described in Published PCT Application WO 98/17336. The syringe comprises a plurality of cooperating attachment members over a range of axial positions on at least one of the plunger extension or the powered drive member. Accordingly, the operative attachment of the plunger and the drive member is possible at multiple axial positions of the plunger for different axial positions of the drive member relative to the plunger.

As compared to syringes and insulin pens, pump type delivery devices can be significantly more convenient to a patient, in that accurate doses of insulin may be calculated and delivered automatically to a patient at any time during the day or night. Furthermore, when used in conjunction with glucose sensors or monitors, insulin pumps may be automatically controlled to provide appropriate doses of an infusion medium at appropriate times of need, based on sensed or monitored levels of blood glucose. As a result, pump type delivery devices have become an important aspect of modem medical treatments of various types of medical conditions, such as diabetes, and the like. As pump technologies improve and doctors and patients become more familiar with such devices, external medical infusion pump treatments are expected to increase in popularity and are expected to increase substantially in number over the next decade.

### SUMMARY OF THE DISCLOSURE

Embodiments of the present invention relate to systems and methods that allow for reservoir filling. Some embodiments of the present invention allow for delivering an infusion medium from a reservoir to the body of a patient.

A system in accordance with an embodiment of the present invention includes a reservoir, a piston, a plunger shaft, and a handle. The reservoir allows for holding an infusion medium. The piston is disposed at least partially within the reservoir, and the piston is moveable to allow the infusion medium to fill into the reservoir and to force the infusion medium out of the reservoir. The plunger shaft is connected to the piston. The plunger shaft has a mating portion for mating with a linkage portion of a drive device, where the drive device allows for driving the plunger shaft so as to move the piston to force the infusion medium out of the reservoir when the linkage portion of the drive device is mated with the mating portion of the plunger shaft. The handle has a handle mating portion for mating with the mating portion of the plunger shaft. The handle is capable of being used by a user to move the plunger shaft so as to move the piston to allow the infusion medium to fill into the reservoir when the handle mating portion of the handle is mated with the mating portion of the plunger shaft.

In various embodiments, the mating portion of the plunger shaft is threaded. Also, in various embodiments, the handle mating portion of the handle is threaded. In some embodiments, the mating portion of the plunger shaft includes a partial nut, and the handle mating portion of the handle includes a threaded interface. Also, in some embodiments, the handle has a gripping arm for gripping the plunger shaft when the handle mating portion of the handle is mated with the mating portion of the plunger shaft.

In various embodiments, the reservoir has a port that is connectable to an infusion path to allow for delivering the infusion medium from the reservoir to the body of a particular user. In further embodiments, the port is connectable to a transfer path to allow for the infusion medium to be filled into the reservoir from an infusion medium container. In various embodiments, the system further includes a transfer guard that is connectable to the reservoir for providing a path to allow the infusion medium to be transferred from an infusion medium container to the reservoir.

In some embodiments, the system further includes a base adapted to be secured to a particular user, and the reservoir is connected to the base. In further embodiments, the reservoir is connectable to an infusion path to allow for delivering the infusion medium from the reservoir to the body of the particular user through an opening in the base. Also, in some embodiments, the system further includes the drive device having the linkage portion, where the drive device further includes a motor for moving the linkage portion, and where the motor is able to move the linkage portion of the drive device so as to drive the plunger shaft when the linkage portion of the drive device is mated with the mating portion of the plunger shaft.

In various embodiments, the linkage portion of the drive device is threaded. Also, in various embodiments, the system includes a disposable housing for housing the reservoir and for being secured to a particular user, and a durable housing for housing the motor of the drive device, where the durable housing is configured to be selectively engaged with and disengaged from the disposable housing. In some embodiments, the reservoir has a degassing portion that includes a hydrophobic material for allowing gases to escape from the reservoir while keeping the infusion medium within the reservoir. Also, in some embodiments, the piston has a degassing portion that includes a hydrophobic material for allowing gases to escape from the reservoir while keeping the infusion medium within the reservoir. In various embodiments, the system may include a particular degassing portion that is located anywhere in a fluid line. Also, in various embodiments, the system may include a hydrophobic material that is able to be sealed after it has been used for degassing.

### BRIEF DESCRIPTION OF THE DRAWINGS

FIG. 1 illustrates a generalized representation of an infusion medium delivery system in accordance with an embodiment of the present invention;

FIG. 2 illustrates an example of an infusion medium delivery system in accordance with an embodiment of the present invention;

FIG. 3 illustrates an example of a delivery device in accordance with an embodiment of the present invention;

FIG. 4 illustrates a view of a delivery device in accordance with an embodiment of the present invention;

FIG. 5A illustrates a durable portion of a delivery device in accordance with an embodiment of the present invention;

FIG. 5B illustrates a section view of a durable portion of a delivery device in accordance with an embodiment of the present invention;

FIG. 5C illustrates a section view of a durable portion of a delivery device in accordance with an embodiment of the present invention;

FIG. 6A illustrates a disposable portion of a delivery device in accordance with an embodiment of the present invention;

FIG. 6B illustrates a section view of a disposable portion of a delivery device in accordance with an embodiment of the present invention;

FIG. 6C illustrates a section view of a disposable portion of a delivery device in accordance with an embodiment of the present invention;

FIG. 7 illustrates a block diagram of a system in accordance with an embodiment of the present invention;

FIG. 8 illustrates a portion of an embodiment of a system in accordance with an embodiment of the present invention;

FIG. 9 illustrates a portion of an embodiment of a system in accordance with another embodiment of the present invention;

FIG. 10 illustrates another portion of an embodiment of a system in accordance with an embodiment of the present invention;

FIG. 11 illustrates a portion of an embodiment of a system in accordance with an embodiment of the present invention;

FIG. 12 illustrates another portion of an embodiment of a system in accordance with an embodiment of the present invention;

FIG. 13 illustrates a portion of an embodiment of a system in accordance with an embodiment of the present invention;

FIG. 14 illustrates a flow chart of a method of using an embodiment of a system in accordance with an embodiment of the present invention;

### DETAILED DESCRIPTION OF PREFERRED EMBODIMENTS

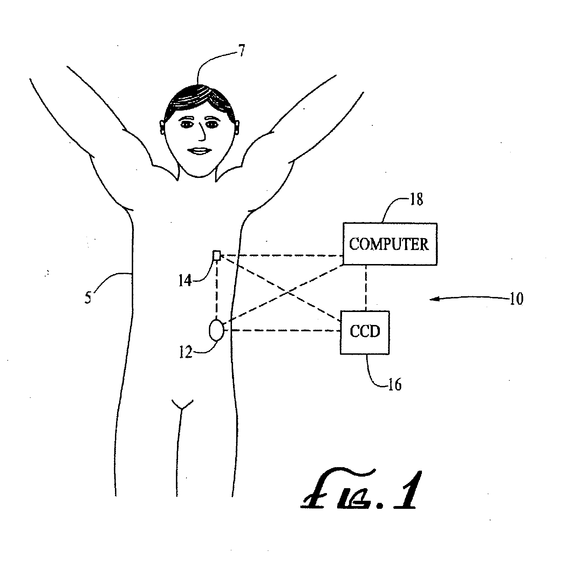

FIG. 1 illustrates a generalized representation of an infusion medium delivery system 10 in accordance with an embodiment of the present invention. The infusion medium delivery system 10 includes a delivery device 12. The infusion medium delivery system 10 may further include a sensing device 14, a command control device (CCD) 16, and a computer 18. In various embodiments, the delivery device 12 and the sensing device 14 may be secured at desired locations on the body 5 of a patient or user 7. The locations at which the delivery device 12 and the sensing device 14 are secured to the body 5 of the user 7 in FIG. 1 are provided only as representative, non-limiting, examples.

The delivery device 12 is configured to deliver an infusion medium to the body 5 of the user 7. In various embodiments, the infusion medium includes a liquid, a fluid, a gel, or the like. In some embodiments, the infusion medium includes a medicine or a drug for treating a disease or a medical condition. For example, the infusion medium may include insulin for treating diabetes, or may include a drug for treating pain, cancer, a pulmonary disorder, HIV, or the like. In some embodiments, the infusion medium includes a nutritional supplement, a dye, a tracing medium, a saline medium, a hydration medium, or the like.

The sensing device 14 includes a sensor, a monitor, or the like, for providing sensor data or monitor data. In various embodiments, the sensing device 14 may be configured to sense a condition of the user 7. For example, the sensing device 14 may include electronics and enzymes reactive to a biological condition, such as a blood glucose level, or the like, of the user 7. In various embodiments, the sensing device 14 may be secured to the body 5 of the user 7 or embedded in the body 5 of the user 7 at a location that is remote from the location at which the delivery device 12 is secured to the body 5 of the user 7. In various other embodiments, the sensing device 14 may be incorporated within the delivery device 12.

Each of the delivery device 12, the sensing device 14, the CCD 16, and the computer 18 may include transmitter, receiver, or transceiver electronics that allow for communication with other components of the infusion medium delivery system 10. The sensing device 14 may be configured to transmit sensor data or monitor data to the delivery device 12. The sensing device 14 may also be configured to communicate with the CCD 16. The delivery device 12 may include electronics and software that are configured to analyze sensor data and to deliver the infusion medium to the body 5 of the user 7 based on the sensor data and/or preprogrammed delivery routines.

The CCD 16 and the computer 18 may include electronics and other components configured to perform processing, delivery routine storage, and to control the delivery device 12. By including control functions in the CCD 16 and/or the computer 18, the delivery device 12 may be made with more simplified electronics. However, in some embodiments, the delivery device 12 may include all control functions, and may operate without the CCD 16 and the computer 18. In various embodiments, the CCD 16 may be a portable electronic device. Also, in various embodiments, the delivery device 12 and/or the sensing device 14 may be configured to transmit data to the CCD 16 and/or the computer 18 for display or processing of the data by the CCD 16 and/or the computer 18. Examples of the types of communications and/or control capabilities, as well as device feature sets and/or program options may be found in U.S. Patent Application Serial No. 10/445,477 filed May 27, 2003, and entitled "External Infusion Device with Remote Programming, Bolus Estimator and/or Vibration Alarm Capabilities," and U.S. Patent Application Serial No. 10/429,385 filed May 5, 2003, and entitled "Handheld Personal Data Assistant (PDA) with a Medical Device and Method of Using the Same," U.S. Patent Application Serial No. 09/813,660 filed March 21, 2001, and entitled "Control Tabs For Infusion Devices And Methods Of Using The Same".

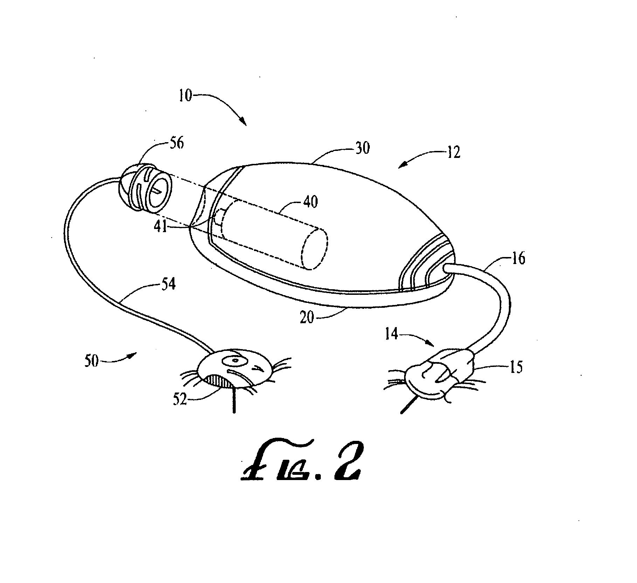

FIG. 2 illustrates an example of the infusion medium delivery system 10 in accordance with an embodiment of the present invention. The infusion medium delivery system 10 in accordance with the embodiment illustrated in FIG. 2 includes the delivery device 12 and the sensing device 14. The delivery device 12 in accordance with an embodiment of the present invention includes a disposable housing 20, a durable housing 30, and a reservoir 40. The delivery device 12 may further include an infusion path 50.

Elements of the delivery device 12 that ordinarily contact the body of a user or that ordinarily contact an infusion medium during operation of the delivery device 12 may be considered as a disposable portion of the delivery device 12. For example, a disposable portion of the delivery device 12 may include the disposable housing 20 and the reservoir 40. The disposable portion of the delivery device 12 may be recommended for disposal after a specified number of uses.

On the other hand, elements of the delivery device 12 that do not ordinarily contact the body of the user or the infusion medium during operation of the delivery device 12 may be considered as a durable portion of the delivery device 12. For example, a durable portion of the delivery device 12 may include the durable housing 30, electronics (not shown in FIG. 2), a drive device having a motor and drive linkage (not shown in FIG. 2), and the like. Elements of the durable housing portion of the delivery device 12 are typically not contaminated from contact with the user or the infusion medium during normal operation of the delivery device 12 and, thus, may be retained for re-use with replaced disposable portions of the delivery device 12.

In various embodiments, the disposable housing 20 supports the reservoir 40 and has a bottom surface (facing downward and into the page in FIG. 2) that is configured to secure to the body of a user. An adhesive may be employed at an interface between the bottom surface of the disposable housing 20 and the skin of a user, so as to adhere the disposable housing 20 to the skin of the user. In various embodiments, the adhesive may be provided on the bottom surface of the disposable housing 20, with a peelable cover layer covering the adhesive material. In this manner, the cover layer may be peeled off to expose the adhesive material, and the adhesive side of the disposable housing 20 may be placed against the skin of the user.

The reservoir 40 is configured for containing or holding an infusion medium, such as, but not limited to insulin. In various embodiments, the reservoir 40 includes a hollow interior volume for receiving the infusion medium, such as, but not limited to, a cylinder-shaped volume, a tubular-shaped volume, or the like. In some embodiments, the reservoir 40 may be provided as a cartridge or canister for containing an infusion medium. In various embodiments, the reservoir 40 is able to be refilled with an infusion medium.

The reservoir 40 may be supported by the disposable housing 20 in any suitable manner. For example, the disposable housing 20 may be provided with projections or struts (not shown), or a trough feature (not shown), for holding the reservoir 40. In some embodiments, the reservoir 40 may be supported by the disposable housing 20 in a manner that allows the reservoir 40 to be removed from the disposable housing 20 and replaced with another reservoir. Alternatively, or in addition, the reservoir 40 may be secured to the disposable housing 20 by a suitable adhesive, a strap, or other coupling structure.

In various embodiments, the reservoir 40 includes a port 41 for allowing an infusion medium to flow into and/or flow out of the interior volume of the reservoir 40. In some embodiments, the infusion path 50 includes a connector 56, a tube 54, and a needle apparatus 52. The connector 56 of the infusion path 50 may be connectable to the port 41 of the reservoir 40. In various embodiments, the disposable housing 20 is configured with an opening near the port 41 of the reservoir 40 for allowing the connector 56 of the infusion path 50 to be selectively connected to and disconnected from the port 41 of the reservoir 40.

In various embodiments, the port 41 of the reservoir 40 is covered with or supports a septum (not shown in FIG. 2), such as a self-sealing septum, or the like. The septum may be configured to prevent an infusion medium from flowing out of the reservoir 40 through the port 41 when the septum is not pierced. Also, in various embodiments, the connector 56 of the infusion path 50 includes a needle for piercing the septum covering the port 41 of the reservoir 40 so as to allow the infusion medium to flow out of the interior volume of the reservoir 40. Examples of needle/septum connectors can be found in U.S. Patent Application Serial No. 10/328,393 filed December 22, 2003, and entitled "Reservoir Connector," which is incorporated herein by reference in its entirety. In other alternatives, non-septum connectors such as Luer locks, or the like may be used. In various embodiments, the needle apparatus 52 of the infusion path 50 includes a needle that is able to puncture skin of a user. Also, in various embodiments, the tube 54 connects the connector 56 with the needle apparatus 52 and is hollow, such that the infusion path 50 is able to provide a path to allow for the delivery of an infusion medium from the reservoir 40 to the body of a user.

The durable housing 30 of the delivery device 12 in accordance with various embodiments of the present invention includes a housing shell configured to mate with and secure to the disposable housing 20. The durable housing 30 and the disposable housing 20 may be provided with correspondingly shaped grooves, notches, tabs, or other suitable features, that allow the two parts to easily connect together, by manually pressing the two housings together, by twist or threaded connection, or other suitable manner of connecting the parts that is well known in the mechanical arts. In various embodiments, the durable housing 30 and the disposable housing 20 may be connected to each other using a twist action. The durable housing 30 and the disposable housing 20 may be configured to be separable from each other when a sufficient force is applied to disconnect the two housings from each other. For example, in some embodiments the disposable housing 20 and the durable housing 30 may be snapped together by friction fitting. In various embodiments, a suitable seal, such as an o-ring seal, may be placed along a peripheral edge of the durable housing 30 and/or the disposable housing 20, so as to provide a seal against water entering between the durable housing 30 and the disposable housing 20.

The durable housing 30 of the delivery device 12 may support a drive device (not shown in FIG. 2), including a motor and a drive device linkage portion, for applying a force to the infusion medium within the reservoir 40 to force the infusion medium out of the reservoir 40 and into an infusion path, such as the infusion path 50, for delivery to a user. For example, in some embodiments, an electrically driven motor may be mounted within the durable housing 30 with appropriate linkage for operatively coupling the motor to a plunger shaft (not shown in FIG. 2) connected to a piston (not shown in FIG. 2) that is within the reservoir 40 and to drive the piston in a direction to force the infusion medium out of the port 41 of the reservoir 40 and to the user. Also, in some embodiments, the motor may be controllable to reverse direction so as to move the plunger shaft and the piston to cause fluid to be drawn into the reservoir 40 from a patient. The motor may be arranged within the durable housing 30 and the reservoir 40 may be correspondingly arranged on the disposable housing 20, such that the operable engagement of the motor with the piston, through the appropriate linkage, occurs automatically upon the user connecting the durable housing 30 with the disposable housing 20 of the delivery device 12. Further examples of linkage and control structures may be found in U.S. Patent Application Serial No. 09/813,660 filed March 21, 2001, and entitled "Control Tabs For Infusion Devices And Methods Of Using The Same," which is incorporated herein by reference in its entirety.

In various embodiments, the durable housing 30 and the disposable housing 20 may be made of suitably rigid materials that maintain their shape, yet provide sufficient flexibility and resilience to effectively connect together and disconnect, as described above. The material of the disposable housing 20 may be selected for suitable compatibility with skin. For example, the disposable housing 20 and the durable housing 30 of the delivery device 12 may be made of any suitable plastic, metal, composite material, or the like. The disposable housing 20 may be made of the same type of material or a different material relative to the durable housing 30. In some embodiments, the disposable housing 20 and the durable housing 30 may be manufactured by injection molding or other molding processes, machining processes, or combinations thereof.

For example, the disposable housing 20 may be made of a relatively flexible material, such as a flexible silicone, plastic, rubber, synthetic rubber, or the like. By forming the disposable housing 20 of a material capable of flexing with the skin of a user, a greater level of user comfort may be achieved when the disposable housing 20 is secured to the skin of the user. Also, a flexible disposable housing 20 may result in an increase in site options on the body of the user at which the disposable housing 20 may be secured.

In the embodiment illustrated in FIG. 2, the delivery device 12 is connected to the sensing device 14 through a connection element 16 of the sensing device 14. The sensing device 14 may include a sensor 15 that includes any suitable biological or environmental sensing device, depending upon a nature of a treatment to be administered by the delivery device 12. For example, in the context of delivering insulin to a diabetes patient, the sensor 15 may include a blood glucose sensor, or the like.

The sensor 15 may be an external sensor that secures to the skin of a user or, in other embodiments, may be an implantable sensor that is located in an implant site within the body of the user. In further alternatives, the sensor may be included with as a part or along side the infusion cannula and/or needle, such as for example as shown in U.S. Patent Serial No. 11/149,119 filed June 8, 2005, and entitled "Dual Insertion Set," which is incorporated herein by reference in its entirety. In the illustrated example of FIG. 2, the sensor 15 is an external sensor having a disposable needle pad that includes a needle for piercing the skin of the user and enzymes and/or electronics reactive to a biological condition, such as blood glucose level or the like, of the user. In this manner, the delivery device 12 may be provided with sensor data from the sensor 15 secured to the user at a site remote from the location at which the delivery device 12 is secured to the user.

While the embodiment shown in FIG. 2 includes a sensor 15 connected by the connection element 16 for providing sensor data to sensor electronics (not shown in FIG. 2) located within the durable housing 30 of the delivery device 12, other embodiments may employ a sensor 15 located within the delivery device 12. Yet other embodiments may employ a sensor 15 having a transmitter for communicating sensor data by a wireless communication link with receiver electronics (not shown in FIG. 2) located within the durable housing 30 of the delivery device 12. In various embodiments; a wireless connection between the sensor 15 and the receiver electronics within the durable housing 30 of the delivery device 12 may include a radio frequency (RF) connection, an optical connection, or another suitable wireless communication link. Further embodiments need not employ the sensing device 14 and, instead, may provide infusion medium delivery functions without the use of sensor data.

As described above, by separating disposable elements of the delivery device 12 from durable elements, the disposable elements may be arranged on the disposable housing 20, while durable elements may be arranged within a separable durable housing 30. In this regard, after a prescribed number of uses of the delivery device 12, the disposable housing 20 may be separated from the durable housing 30, so that the disposable housing 20 may be disposed of in a proper manner. The durable housing 30 may then be mated with a new (unused) disposable housing 20 for further delivery operation with a user.

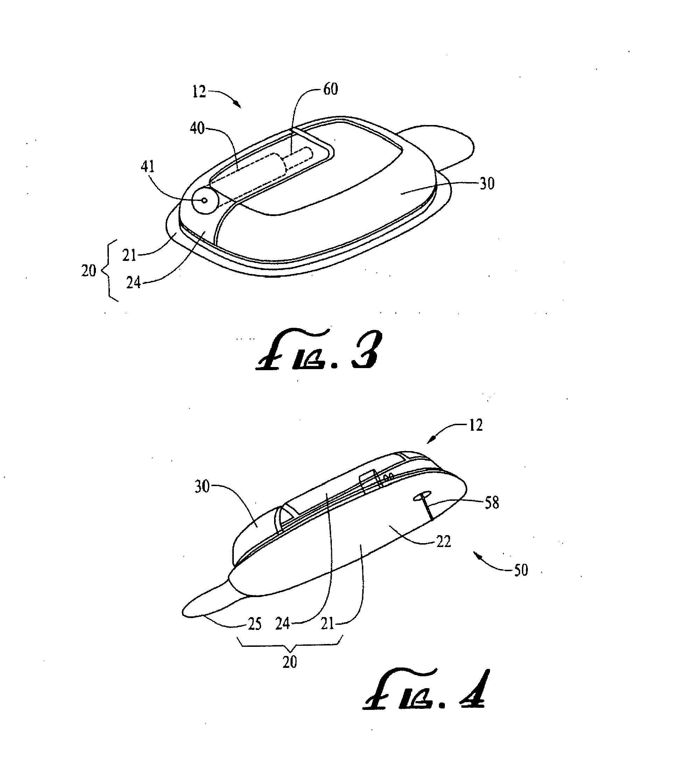

FIG. 3 illustrates an example of the delivery device 12 in accordance with another embodiment of the present invention. The delivery device 12 of the embodiment of FIG. 3 is similar to the delivery device 12 of the embodiment of FIG. 2. While the delivery device 12 in the embodiment illustrated in FIG. 2 provides for the durable housing 30 to cover the reservoir 40, the delivery device 12 in the embodiment of FIG. 3 provides for the durable housing 30 to secure to the disposable housing 20 without covering the reservoir 40. The delivery device 12 of the embodiment illustrated in FIG. 3 includes the disposable housing 20, and the disposable housing 20 in accordance with the embodiment illustrated in FIG. 3 includes a base 21 and a reservoir retaining portion 24. In one embodiment, the base 21 and reservoir retaining portion 24 may be formed as a single, unitary structure.

The base 21 of the disposable housing 20 is configured to be secured to the body of a user. The reservoir retaining portion 24 of the disposable housing 20 is configured to house the reservoir. The reservoir retaining portion 24 of the disposable housing 20 may be configured to have an opening to allow for the port 41 of the reservoir 40 to be accessed from outside of the reservoir retaining portion 24 while the reservoir 40 is housed in the reservoir retaining portion 24. The durable housing 30 may be configured to be attachable to and detachable from the base 21 of the disposable housing 20. The delivery device 12 in the embodiment illustrated in FIG. 3 includes a plunger shaft 60 that is connected to or that is connectable to a piston (not shown in FIG. 3) within the reservoir 40.

FIG. 4 illustrates another view of the delivery device 12 of the embodiment of FIG. 3. The delivery device 12 of the embodiment illustrated in FIG. 4 includes the disposable housing 20, the durable housing 30, and the infusion path 50. The disposable housing 20 in the embodiment of FIG. 4 includes the base 21, the reservoir retaining portion 24, and a peelable cover layer 25. The peelable cover layer 25 may cover an adhesive material on the bottom surface 22 of the base 21. The peelable cover layer 25 may be configured to be peelable by a user to expose the adhesive material on the bottom surface 22 of the base 21. In some embodiments, there may be multiple adhesive layers on the bottom surface 22 of the base 21 that are separated by peelable layers.

The infusion path 50 in accordance with the embodiment of the present invention illustrated in FIG. 4 includes the needle 58 rather than the connector 56, the tube 54, and the needle apparatus 52 as shown in the embodiment of FIG. 2. The base 21 of the disposable housing 20 may be provided with an opening or pierceable wall in alignment with a tip of the needle 58, to allow the needle 58 to pass through the base 21 and into the skin of a user under the base 21, when extended. In this manner, the needle 58 may be used to pierce the skin of the user and deliver an infusion medium to the user.

Alternatively, the needle 58 may be extended through a hollow cannula (not shown in FIG. 4), such that upon piercing the skin of the user with the needle 58, an end of the hollow cannula is guided through the skin of the user by the needle 58. Thereafter, the needle 58 may be removed, leaving the hollow cannula in place, with one end of the cannula located within the body of the user and the other end of the cannula in fluid flow connection with the infusion medium within the reservoir 40, to convey pumped infusion media from the reservoir 40 to the body of the user.

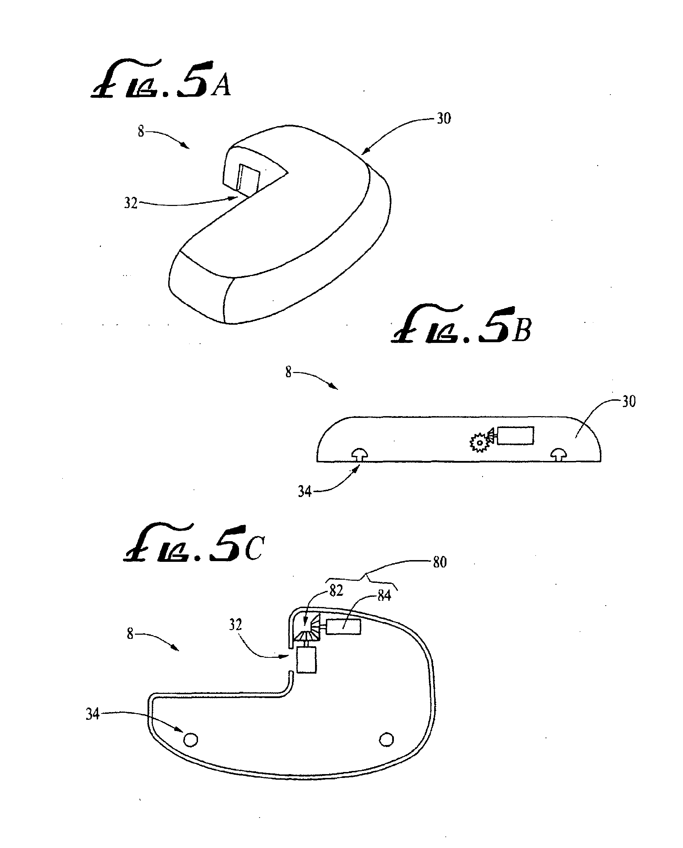

FIG. 5A illustrates a durable portion 8 of the delivery device 12 (refer to FIG. 3) in accordance with an embodiment of the present invention. FIG. 5B illustrates a section view of the durable portion 8 in accordance with an embodiment of the present invention. FIG. 5C illustrates another section view of the durable portion 8 in accordance with an embodiment of the present invention. With reference to FIGs. 5A, 5B, and 5C, in various embodiments, the durable portion 8 includes the durable housing 30, and a drive device 80. The drive device 80 includes a motor 84 and a drive device linkage portion 82. In various embodiments, the durable housing 30 may include an interior volume for housing the motor 84, the drive device linkage portion 82, other electronic circuitry, and a power source (not shown in FIGs. 5A, 5B, and 5C). Also, in various embodiments, the durable housing 30 is configured with an opening 32 for receiving a plunger shaft 60 (refer to FIG. 3). Also, in various embodiments, the durable housing 30 may include one or more connection members 34, such as tabs or the like, for connecting with the base 21 of the disposable housing 20 (refer to FIG. 3).

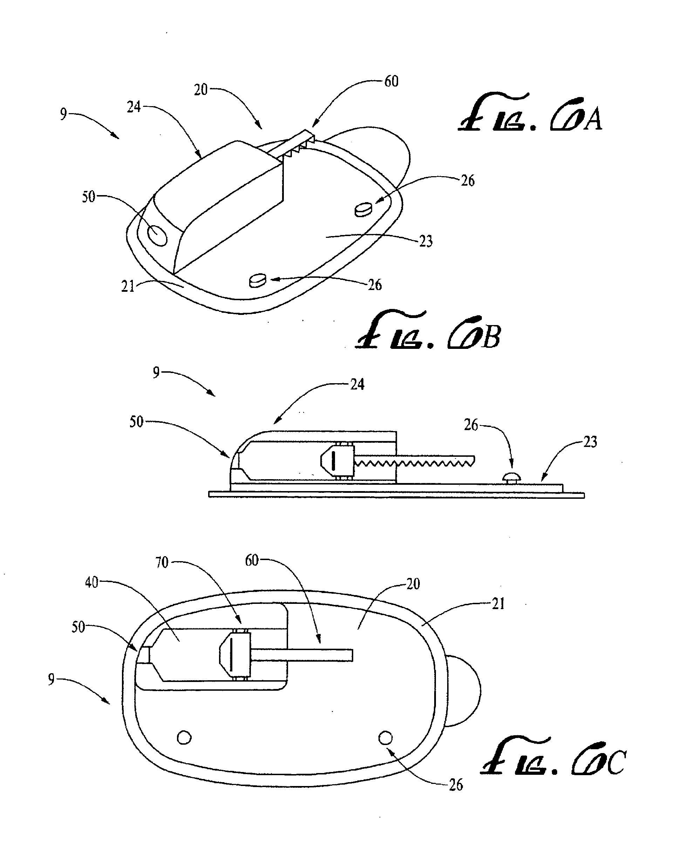

FIG. 6A illustrates a disposable portion 9 of the delivery device 12 (refer to FIG. 3) in accordance with an embodiment of the present invention. FIG. 6B illustrates a section view of the disposable portion 9 in accordance with an embodiment of the present invention. FIG. 6C illustrates another section view of the disposable portion 9 in accordance with an embodiment of the present invention. With reference to FIGs. 6A, 6B, and 6C, in various embodiments, the disposable portion 9 includes the disposable housing 20, the reservoir 40, the plunger shaft 60, and a piston 70. In some embodiments, the disposable housing 20 includes the base 21 and the reservoir retaining portion 24. In various embodiments, the base 21 includes a top surface 23 having one or more connection members 26, such as grooves or the like, for allowing connections with the one or more connection members 34 of embodiments of the durable housing 30 (refer to FIG. 5B).

In various embodiments, the reservoir 40 is housed within the reservoir retaining portion 24 of the disposable housing 20, and the reservoir 40 is configured to hold an infusion medium. Also, in various embodiments, the piston 70 is disposed at least partially within the reservoir 40 and is moveable within the reservoir 40 to allow the infusion medium to fill into the reservoir 40 and to force the infusion medium out of the reservoir 40.

In some embodiments, the plunger shaft 60 is connected to or is connectable to the piston 70. Also, in some embodiments, a portion of the plunger shaft 60 extends to outside of the reservoir retaining portion 24 of the disposable housing 20. In various embodiments, the plunger shaft 60 has a mating portion for mating with the drive device linkage portion 82 of the drive device 80 (refer to FIG. 5C). With reference to FIGs. 5C and 6C, in some embodiments, the durable housing 30 may be snap fitted onto the disposable housing 20, whereupon the drive device linkage portion 82 automatically engages the mating portion of the plunger shaft 60.

When the durable housing 30 and the disposable housing 20 are fitted together with the drive device linkage portion 82 engaging or mating with the plunger shaft 60, the motor 84 may be controlled to drive the drive device linkage portion 82 and, thus, move the plunger shaft 60 to cause the piston 70 to move within the reservoir 40. When the interior volume of the reservoir 40 is filled with an infusion medium and an infusion path 50 is provided from the reservoir 40 to the body of a user, the piston 70 may be moved within the reservoir 40 to force the infusion medium from the reservoir 40 and into the infusion path 50, so as to deliver the infusion medium to the body of the user.

In various embodiments, once the reservoir 40 has been sufficiently emptied or otherwise requires replacement, a user may simply remove the durable housing 30 from the disposable housing 20, and replace the disposable portion 9, including the reservoir 40, with a new disposable portion having a new reservoir. The durable housing 30 may be connected to the new disposable housing of the new disposable portion, and the delivery device including the new disposable portion may be secured to the skin of a user. In various other embodiments, rather than replacing the entire disposable portion 9 every time the reservoir 40 is emptied, the reservoir 40 may be refilled with an infusion medium. In some embodiments, the reservoir 40 may be refilled while remaining within the reservoir retaining portion 24 (refer to FIG. 6B) of the disposable housing 20. Also, in various embodiments, the reservoir 40 may be replaced with a new reservoir (not shown), while the disposable housing 20 may be re-used with the new reservoir. In such embodiments, the new reservoir may be inserted into the disposable portion 9.

With reference to FIGs. 3, 5A, and 6B, in various embodiments, the delivery device 12 includes reservoir status circuitry (not shown), and the reservoir 40 includes reservoir circuitry (not shown). In various embodiments, the reservoir circuitry stores information such as, but not limited to, at least one of (i) an identification string identifying the reservoir 40; (ii) a manufacturer of the reservoir 40; (iii) contents of the reservoir 40; and (iv) an amount of contents in the reservoir 40. In some embodiments, the delivery device 12 includes the reservoir status circuitry (not shown), and the reservoir status circuitry is configured to read data from the reservoir circuitry when the reservoir 40 is inserted into the disposable portion 9. In various embodiments, the reservoir status circuitry is further configured to store data to the reservoir circuitry after at least some of the contents of the reservoir 40 have been transferred out of the reservoir 40, so as to update information in the reservoir circuitry related to an amount of contents still remaining in the reservoir 40. In some embodiments, the reservoir status circuitry is configured to store data to the reservoir circuitry, so as to update information in the reservoir Circuitry related to an amount of contents still remaining in the reservoir 40, when the reservoir 40 is inserted into the disposable portion 9. In some embodiments, the delivery device 12 includes the reservoir status circuitry (not shown) and the reservoir 40 includes the reservoir circuitry (not shown), and the reservoir status circuitry selectively inhibits use of the delivery device 12 or selectively provides a warning signal based on information read by the reservoir status circuitry from the reservoir circuitry.

Various systems, structures, and methods allowing for reservoir filling will now be discussed in more detail.

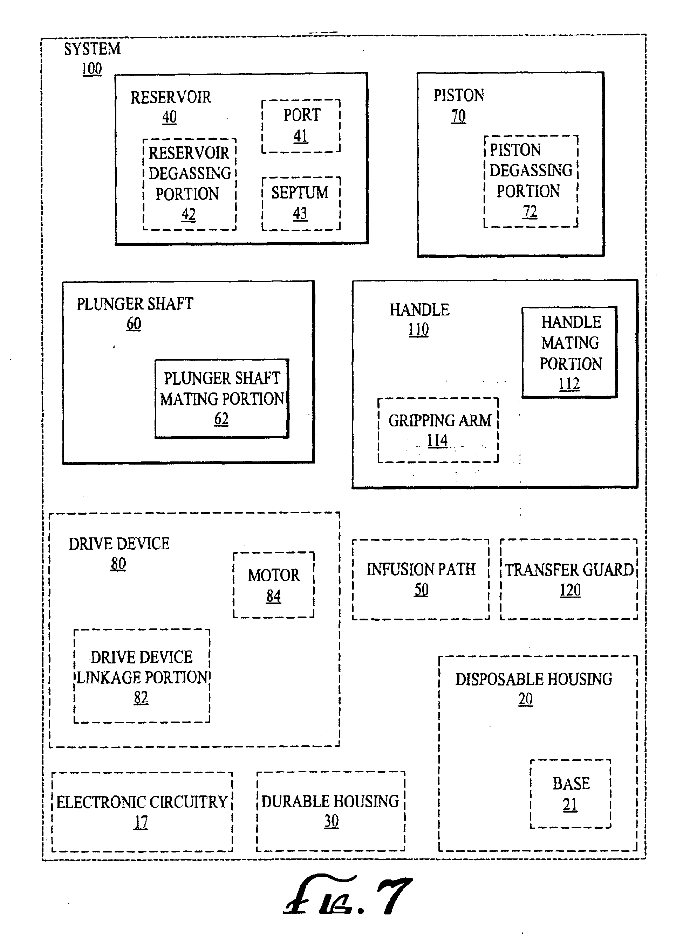

FIG. 7 illustrates a block diagram of a system 100 in accordance with an embodiment of the present invention. In various embodiments, the system 100 includes a reservoir filling system that allows for filling a reservoir, or the like. Also, in various embodiments, the system 100 includes a delivery device, such as the delivery device 12, or the like. In some embodiments, the system 100 includes an infusion medium delivery system, such as the infusion medium delivery system 10, or the like. In the embodiment illustrated in FIG. 7, the system 100 includes the reservoir 40, the piston 70, the plunger shaft 60, and a handle 110. Also, in the embodiment illustrated in FIG. 7, the plunger shaft 60 includes a plunger shaft mating portion 62, and the handle 110 includes a handle mating portion 112.

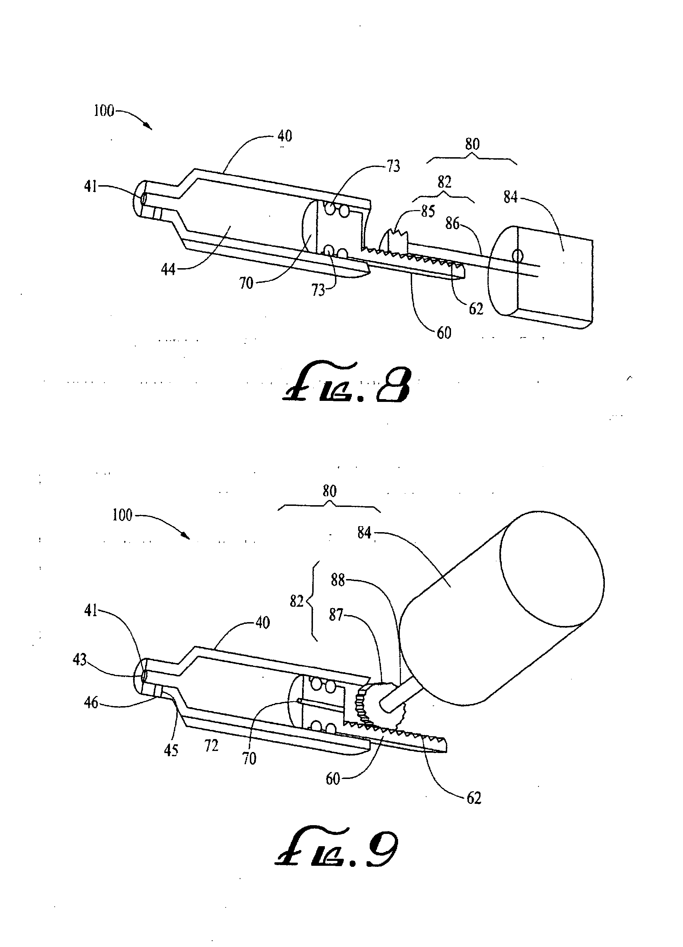

In various embodiments, the reservoir 40 is configured to hold an infusion medium. In various embodiments, the reservoir 40 is made of, for example, a suitable metal, plastic, ceramic, glass, composite material, or the like. In some embodiments, the reservoir 40 includes a canister or the like. Also, in some embodiments, the reservoir 40 has a hollow interior for containing or holding the infusion medium. The reservoir 40 may have a port 41 (refer to FIGs. 7 and 8) that allows for the infusion medium to flow into and/or out of the reservoir 40.

In some embodiments, the reservoir 40 includes a septum 43 (refer to FIGs. 7 and 9) that is positioned within an opening defined by the port 41 of the reservoir 40, where the septum 43 is capable of being pierced to allow the infusion medium to flow into the reservoir 40, and where the septum 43 is capable of holding the infusion medium within the reservoir 40 when the septum 43 is not pierced. In various embodiments, the septum 43 may be compressed around a needle (not shown in FIG. 7) that pierces the septum 43 for sealing against the needle and may be a self-sealing septum that re-seals itself, after removal of the needle. In some embodiments, the septum 43 may be compressed to provide a better seal around the needle. Also, in various embodiments, the septum 43 is formed of a suitable material such as, but not limited to, rubber, silicone rubber, polyurethane, or other materials that may be pierced by a needle and form a seal around the needle.

In various embodiments, the reservoir 40 includes a reservoir degassing portion 42. In such embodiments, the reservoir degassing portion 42 allows for gases to be released from the reservoir 40 while maintaining an infusion medium, such as liquids or the like, within the interior volume of the reservoir 40. In some embodiments, the reservoir degassing portion 42 includes a hydrophobic material or the like that will allow for air and other gases to pass through, but will not allow liquids, such as water, syringe deliverable insulin, or the like, to pass through. Also, in some embodiments, the reservoir degassing portion 42 includes a material such as a hydrophobic membrane, or the like, that is manufactured by Gore^{™}. Such a reservoir degassing portion 42 may be positioned in any suitable position with respect to the reservoir 40 and may extend from an interior surface of the reservoir 40 to an exterior surface of the reservoir 40 to allow for gases to pass from the interior volume of the reservoir 40 to outside of the reservoir 40, so as to allow for degassing the interior volume of the reservoir 40. Examples of further structures that permit air-flow, but that inhibit fluids can be found in U.S. Patent Application Serial No. 10/328,393 filed December 22, 2003, and entitled "Reservoir Connector," and U.S. Patent Application Serial No. 10/699,429 filed October 31, 2003, and entitled "External Infusion Device with a Vented Housing," both of which are incorporated herein by reference in their entirety.

In various embodiments that include the reservoir degassing portion 42, once gases are removed from the interior volume of the reservoir 40 through the reservoir degassing portion 42, the reservoir 40 is sealed to prevent gases from re-entering the reservoir 40 and to prevent evaporation of an infusion medium in the reservoir 40. In some embodiments that include the reservoir degassing portion 42, the reservoir degassing portion 42 is used to degas the reservoir 40 with positive pressure and then is removed or covered to prevent evaporation of an infusion medium that is in the reservoir 40.

In various embodiments, the piston 70 (refer to FIGs. 7, 8, and 9) is disposed at least partially within the reservoir 40, where the piston 70 is moveable to allow the infusion medium to fill into the reservoir 40 and to force the infusion medium out of the reservoir 40. The piston 70 may be made of a suitably rigid material such as, but not limited to, metal, plastic, ceramic, glass, a composite material, or the like. In some embodiments, the piston 70 has a head with an outside diameter of slightly less than the inside diameter of the interior of the reservoir 40. In other embodiments, the piston 70 has a head with an outside diameter of slightly greater than or equal to the inside diameter of the interior of the reservoir 40, and the piston 70 may be compressible to fit within the reservoir 40. In various embodiments, the piston 70 extends partially into the interior of the reservoir 40 from an opposite side of the reservoir 40 relative to the port 41 of the reservoir 40.

In various embodiments, the piston 70 includes a piston degassing portion 72 (refer to FIGs. 7 and 9). In such embodiments, the piston degassing portion 72 allows for gases to be released from the reservoir 40 through an opening in the piston 70 while maintaining an infusion medium, such as liquids or the like, within the interior volume of the reservoir 40. In some embodiments, the piston degassing portion 72 includes a hydrophobic material or the like that will allow for air and other gases to pass through, but will not allow liquids, such as water, syringe deliverable insulin, or the like, to pass through. Also, in some embodiments, the piston degassing portion 72 includes a material such as a hydrophobic membrane, or the like, that is manufactured by Gore^{™}. Examples of structures that permit air-flow, but that inhibit fluids can be found in U.S. Patent Application Serial No. 10/328,393 filed December 22, 2003, and entitled "Reservoir Connector," and U.S. Patent Application Serial No. 10/699,429 filed October 31, 2003, and entitled "External Infusion Device with a Vented Housing," both of which are incorporated herein by reference in their entirety. Such a piston degassing portion 72 may be positioned in any suitable position with respect to the piston 70 and may extend from a first surface of the piston 70 that faces the interior volume of the reservoir 40 to a second surface of the piston 70 that is opposite the first surface, to allow for gases to pass from the interior volume of the reservoir 40 to outside of the reservoir 40, so as to allow for degassing the interior volume of the reservoir 40.

In various embodiments that include the piston degassing portion 72, once gases are removed from the interior volume of the reservoir 40 through the piston degassing portion 72, the piston 70 is sealed to prevent gases from re-entering the reservoir 40 and to prevent evaporation of an infusion medium in the reservoir 40. In some embodiments that include the piston degassing portion 72, the piston degassing portion 72 is used to degas the reservoir 40 with positive pressure and then is removed or covered to prevent evaporation of an infusion medium that is in the reservoir 40.

In various embodiments, the plunger shaft 60 (refer to FIGs. 7, 8, and 9) is connected to the piston 70. In some embodiments, the plunger shaft 60 is formed as a single unit with the piston 70. In various other embodiments, the plunger shaft 60 is attached to the piston 70 by, for example, an adhesive, a screw, joining engagement portions of the plunger shaft 60 and the piston 70, or the like. The plunger shaft 60 may be made of a suitably rigid material such as, but not limited to, metal, plastic, ceramic, glass, a composite material, or the like.

In some embodiments, the plunger shaft 60 includes the plunger shaft mating portion 62. In such embodiments, the plunger shaft mating portion 62 is configured to allow for mating with a linkage portion of a drive device, such as the drive device linkage portion 82 of the drive device 80 (refer to FIGs. 7, 8, and 9). In various embodiments, the drive device 80 allows for driving the plunger shaft 60 so as to move the piston 70 to force the infusion medium out of the reservoir 40 when the drive device linkage portion 82 of the drive device 80 is mated with the plunger shaft mating portion 62 of the plunger shaft 60. In some embodiments, the plunger shaft mating portion 62 is provided with threads, keys, key slots, or the like, that are configured to operatively engage or mate with corresponding threads, keys, key slots, or the like, of the drive device linkage portion 82. In some embodiments, the plunger shaft 60 includes a partial nut, a lead screw, or the like.

The handle 110 includes the handle mating portion 112 (refer to FIGs. 7, 12, 13, and 30). The handle 110 may be made of a suitably rigid material such as, but not limited to, metal, plastic, ceramic, glass, a composite material, or the like. The handle mating portion 112 of the handle 110 is configured to allow for mating with the plunger shaft mating portion 62 of the plunger shaft 60. In some embodiments, the handle mating portion 112 is provided with threads, keys, key slots, or the like, that are configured to operatively engage or mate with corresponding threads, keys, key slots, or the like, of the plunger shaft mating portion 62. In various embodiments, the handle mating portion 112 of the handle 110 includes a lead screw, a partial nut, or the like.

The handle 110 is capable of being used by a user to move the plunger shaft 60 so as to move the piston 70 to allow the infusion medium to flow or fill into the reservoir 40 when the handle mating portion 112 of the handle 110 is mated with the plunger shaft mating portion 62 of the plunger shaft 60. The handle mating portion 112 of the handle 110 may be mated with the plunger shaft mating portion 62 of the plunger shaft 60 when the drive device linkage portion 82 of the drive device 80 has been disconnected or disengaged from the plunger shaft mating portion 62 of the plunger shaft 60.

In various embodiments, the handle 110 further includes a gripping arm 114 for gripping the plunger shaft 60 when the handle mating portion 112 of the handle 110 is mated with the plunger shaft mating portion 62 of the plunger shaft 60. In some embodiments, the plunger shaft mating portion 62 includes a partial nut, the handle mating portion 112 includes a threaded interface, and the gripping arm 114 extends from the handle mating portion 112 of the handle 110. In such embodiments, the handle mating portion 112 may be mated with the plunger shaft mating portion 62 and then rotated to cause the gripping arm 114 to grip the plunger shaft 60 between the gripping arm 114 and the handle mating portion 112 of the handle 110.

In various embodiments, the system 100 further includes the drive device 80 (refer to FIGs. 7, 8, and 9). In some embodiments, the drive device 80 includes the drive device linkage portion 82 and the motor 84. The motor 84 may be mechanically coupled to the drive device linkage portion 82 to drive the drive device linkage portion 82 in a controlled manner. For example, the drive device linkage portion 82 may include a threaded lead screw, and the motor 84 may drive the lead screw in a rotary motion about its longitudinal axis. The drive device linkage portion 82 may include one or more suitable gears, belts, chains, drive shafts, or other linkage structures for coupling to the motor 84. Examples of suitable motors that may be used for the motor 84 include, but are not limited to, a DC motor, a flat or pancake DC motor, a servo motor, a stepper motor, an electronically commutated motor, a rotary piezo-electrically actuator motor, and the like. In some embodiments, the drive device linkage portion 82 is provided with threads, keys, key slots, or the like, that are configured to operatively engage or mate with corresponding threads, keys, key slots, or the like, of the plunger shaft mating portion 62. In various embodiments, the drive device linkage portion 82 includes a lead screw, a partial nut, or the like.

In some embodiments, the system 100 further includes the infusion path 50. In some embodiments, the infusion path 50 includes the connector 56, the tube 54, and the needle apparatus 52 as illustrated in FIG. 2, for connecting to the port 41 of the reservoir 40 and for providing a path to deliver the infusion medium from the reservoir 40 to the body of a user. Also, in various embodiments, the infusion path 50 includes the needle 58 as illustrated in FIG. 4 for providing a path to deliver the infusion medium from the reservoir 40 to the body of the user through an opening in the base of a disposable housing. In various embodiments, the port 41 of the reservoir 40 is connectable to the infusion path 50 to allow for delivering the infusion medium from the reservoir to the body of a particular user, where the port 41 is also connectable to a transfer path, such as a transfer guard 120 or the like, to allow the infusion medium to be transferred into the reservoir 40 from an infusion medium container (not shown in FIG. 7), such as a vial, a canister, or the like.

In various embodiments, the system 100 further includes the transfer device, referred to herein as a transfer guard 120 (refer to FIGs. 7 and 10-13). In some embodiments, the transfer guard 120 is connectable to the port 41 of the reservoir 40 for providing a path to allow the infusion medium to be transferred from an infusion medium container to the reservoir 40. In various embodiments, the transfer guard 120 is configured like one or more of the embodiments of the transfer guard as disclosed in U.S. Patent No. 6,591,876, entitled "Needle Safe Transfer Guard".
In some embodiments, the transfer guard 120 includes a degassing portion (not shown) that allows for the reservoir 40 to be filled with an infusion medium and then allows for the reservoir 40 to be degassed, and then is removed to allow the reservoir 40 to be sealed.

In some embodiments, the system 100 further includes the disposable housing 20. In various embodiments, the disposable housing includes the base 21. Also, in various embodiments, the reservoir 40, the piston 70, and the plunger shaft 60 are supported by the base 21 of the disposable housing 20. In some embodiments, the base 21 of the disposable housing 20 is adapted to be secured to a user, such as with an adhesive, or the like. Also, in some embodiments, the reservoir 40 is connected to the base 21 of the disposable housing 20.

In various embodiments, the system 100 further includes the durable housing 30. In some embodiments, the drive device 80 and electronic circuitry 17 are housed or contained within the durable housing 30. In various embodiments of the system 100, the durable housing 30 and the disposable housing 20 are configured as in the embodiment of the delivery device 12 illustrated in FIG. 2. In various other embodiments of the system 100, the durable housing 30 and the disposable housing 20 are configured as in the embodiment of the delivery device 12 illustrated in FIG. 3.

In some embodiments, the system 100 further includes the electronic circuitry 17. In various embodiments, the electronic circuitry 17 may be configured to control the motor 84 according to a desired infusion medium delivery program or profile. A delivery program or profile may be stored within a suitable electronic storage medium (not shown) located within the durable housing 30 and/or may be communicated to the electronic circuitry 17 from other sources, such as the CCD 16 or the sensing device 14 or the computer 18 shown in FIG. 1. Alternatively, or in addition, the electronic circuitry 17 may control the motor 84 to deliver one or more discrete volumes of the infusion medium in response to delivery demand control signals generated within the system 100 or communicated to the system 100 from other sources. In various embodiments, the electronic circuitry 17 may be housed or contained within the durable housing 30.

FIG. 8 illustrates a portion of an embodiment of the system 100 in accordance with an embodiment of the present invention. In the embodiment illustrated in FIG. 8, the reservoir 40 has the port 41, and the piston 70 is disposed within the reservoir 40. In various embodiments, the reservoir 40 defines an infusion medium retaining interior volume or portion 44 for holding or containing an infusion medium. In some embodiments, one or more seals 73 may be provided around an outer peripheral surface of the piston 70, to inhibit a passage of the infusion medium across the piston 70 from the infusion medium retaining interior portion 44 of the reservoir 40 to outside of the reservoir 40.

In various embodiments, the seals 73 may include one or more o-ring seals or other suitable seal structures and may be made of any suitable material, including but not limited to, rubber, silicone rubber, polyurethane or other plastic material, metal, composite material, or the like. In some embodiments, the seals 73 may provide sufficient frictional force between the piston 70 and an interior surface of the reservoir 40 to inhibit rotation of the piston 70 with respect to the reservoir 40. Also, in various embodiments, additional structure may be provided to inhibit rotation of the piston 70 with respect to the reservoir 40 including, but not limited to, one or more keys, projections, or shaped portions on the piston 70 that fit within corresponding one or more grooves along a length of the interior surface of the reservoir 40, or vice versa. For example, the interior surface of the reservoir 40 may have a groove, and the piston 70 may have a corresponding projection that fits slidably within the groove, such that the piston 70 is able to slide within the reservoir 40, but is not able to rotate within the reservoir 40. In yet further embodiments, the cross sectional shape of the piston 70 and of the reservoir 40 may be non-circular, such as, but not limited to, oval, to inhibit rotation of the piston 70 with respect to the reservoir 40. Such a non-circular cross-sectional shape of the piston 70 and of the reservoir 40 may also minimize an overall height the piston 70 and the reservoir 40.

In the embodiment illustrated in FIG. 8, the plunger shaft 60 is formed as a single unit with the piston 70. Also, in the embodiment illustrated in FIG. 8, the plunger shaft 60 has the plunger shaft mating portion 62 that includes a partial nut that is threaded along the longitudinal direction of the plunger shaft 60. Moreover, in the embodiment illustrated in FIG. 8, the drive device linkage portion 82 of the drive device 80 includes a rotatably driven drive shaft 86 on which a drive screw 85 is mounted. In various embodiments, the drive screw 85 of the drive device linkage portion 82 is threaded. During operation of the embodiment illustrated in FIG. 8, the drive screw 85 of the drive device linkage portion 82 is arranged to mate with the plunger shaft mating portion 62 and, upon rotation of the drive shaft 86 by the motor 84, the drive screw 85 is rotated to cause a linear movement of the plunger shaft 60 relative to the reservoir 40 and, as a result, the piston 70 is moved within the reservoir 40.

In various embodiments, the reservoir 40 may be supported by the base 21 of the disposable housing 20, while the motor 84 along with the drive shaft 86 and the drive screw 85 may be supported within the durable housing 30. In such embodiments, when the durable housing 30 is removed from the disposable housing 20, the drive screw 85 may be easily disengaged or disconnected from the plunger shaft mating portion 62 of the plunger shaft 60 by simply lifting the drive screw 85 off of the plunger shaft mating portion 62. Also, in various embodiments, the plunger shaft 60 is positioned with respect to the disposable housing 20 such that when the durable housing 30 is connected to the disposable housing 30, the drive screw 85 automatically mates with the plunger shaft mating portion 62 of the plunger shaft 60.

FIG. 9 illustrates a portion of an embodiment of the system 100 in accordance with another embodiment of the present invention. In the embodiment illustrated in FIG. 9, the reservoir 40 has the port 41 and further has a second port 45. In the embodiment of FIG. 9, the septum 43 is located within the port 41, and a second septum 46 is located within the second port 45. In various embodiments, the port 41 is connectable to an embodiment of the infusion path 50 that includes the connector 56, the tube 54, and the needle apparatus 52 (refer to FIG. 2). Also, in various embodiments, the second port 45 is connectable to an embodiment of the infusion path 50 that includes the needle 58 (refer to FIG. 4). In some embodiments, the reservoir 40 is filled with an infusion medium through the port 41 and the infusion medium is forced out of the reservoir 40 through the second port 45. The embodiment of the reservoir 40 illustrated in FIG. 9 may be said to be a multi-port reservoir, because the reservoir 40 has two ports.

In the embodiment illustrated in FIG. 9, the piston 70 is disposed within the reservoir 40, and the plunger shaft 60 is formed as a single unit with the piston 70. In the embodiment illustrated in FIG. 9, the plunger shaft 60 has the plunger shaft mating portion 62 that includes a threaded rack that is threaded along the longitudinal direction of the plunger shaft 60. Moreover, in the embodiment illustrated in FIG. 9, the drive device linkage portion 82 of the drive device 80 includes a rotatably driven drive shaft 88 on which a pinion gear 87 is fixedly mounted for rotation with rotation of the drive shaft 88. During operation of the embodiment illustrated in FIG. 9, the pinion gear 87 of the drive device linkage portion 82 is arranged to mate with the plunger shaft mating portion 62 and, upon rotation of the drive shaft 88 by the motor 84, the pinion gear 87 is rotated to cause a linear movement of the plunger shaft 60 relative to the reservoir 40 and, as a result, the piston 70 is moved within the reservoir 40.

In various embodiments, the reservoir 40 may be supported by the base 21 of the disposable housing 20, while the motor 84 along with the drive shaft 88 and the pinion gear 87 may be supported within the durable housing 30. In such embodiments, when the durable housing 30 is removed from the disposable housing 20, the pinion gear 87 may be easily disengaged or disconnected from the plunger shaft mating portion 62 of the plunger shaft 60 by simply lifting the pinion gear 87 off of the plunger shaft mating portion 62. Also, in various embodiments, the plunger shaft 60 is positioned with respect to the disposable housing 20 such that when the durable housing 30 is connected to the disposable housing 30, the pinion gear 87 automatically mates with the plunger shaft mating portion 62 of the plunger shaft 60.

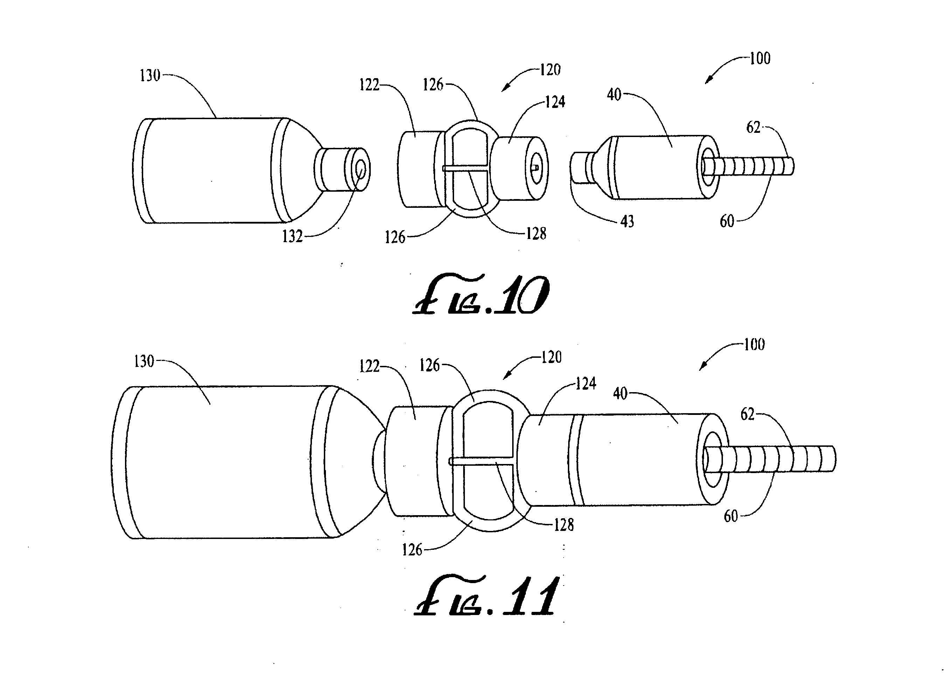

FIG. 10 illustrates another portion of an embodiment of the system 100 in accordance with an embodiment of the present invention. In the embodiment illustrated in FIG. 10, the system 100 includes the reservoir 40, the plunger shaft 60, and the transfer guard 120. In various embodiments, the system 100 further includes an infusion medium container 130. The infusion medium container 130 allows for holding an infusion medium. In various embodiments, the infusion medium container 130 includes a vial, a canister, or the like. Also, in various embodiments, the infusion medium container 130 is made of a suitable material such as, but not limited to, metal, plastic, ceramic, glass, composite material, or the like. In some embodiments, the infusion medium container 130 is configured with an opening, and a septum 132 that is able to be pierced by a needle is located within or over the opening in the infusion medium container 130, and the infusion medium is able to flow out of the opening in the infusion medium container 130 when the septum 132 is pierced.

In various embodiments, the transfer guard 120 includes a supply adapter 122, a receiver adapter 124, a support structure 126, and an infusion medium conducting element 128. In some embodiments, the supply adapter 122 is adapted to be mated with the infusion medium container 130. Also, in some embodiments, the receiver adapter 124 is adapted to be mated with the reservoir 40. In various embodiments, the support structure 126 is coupled between the supply adapter 122 and the receiver adapter 124, where the support structure 126 is configured to allow movement of the supply adapter 122 and the receiver adapter 124 from a first more distant position relative to each other to a second closer position relative to each other.

Also, in various embodiments, the infusion medium conducting element 128 includes a needle or the like that extends from the supply adapter 122 to the receiver adapter 124. In some embodiments, the supply adapter 122 and the receiver adapter 124 are further adapted to substantially protect the corresponding tips of the infusion medium conducting element 128, such as needle tips, from contact with a user. In various embodiments, the infusion medium conducting element 128 is able to pierce the septum 132 of the infusion medium container 130 and is able to pierce the septum 43 of the reservoir 40, so as to establish a transfer path for transferring an infusion medium from the infusion medium container 130 to the reservoir 40.

FIG. 11 illustrates a portion of an embodiment of the system 100 in accordance with the embodiment shown in FIG. 10, where the transfer element 120 has been mated with the infusion medium container 130 and with the reservoir 40. As is illustrated in FIG. 11, in various embodiments, the supply adapter 122 is able to mate with the infusion medium container 130 and the receiver adapter 124 is able to mate with the reservoir 40, such that the infusion medium conducting element provides a transfer path from the infusion medium container 130 to the reservoir 40. In some embodiments, the reservoir 40 includes an opening out of which the plunger shaft 60 extends, where the receiver adapter 124 of the transfer guard 120 is connectable to an opposite side of the reservoir 40 from a side of the reservoir 40 that has the opening out of which the plunger shaft 60 extends.

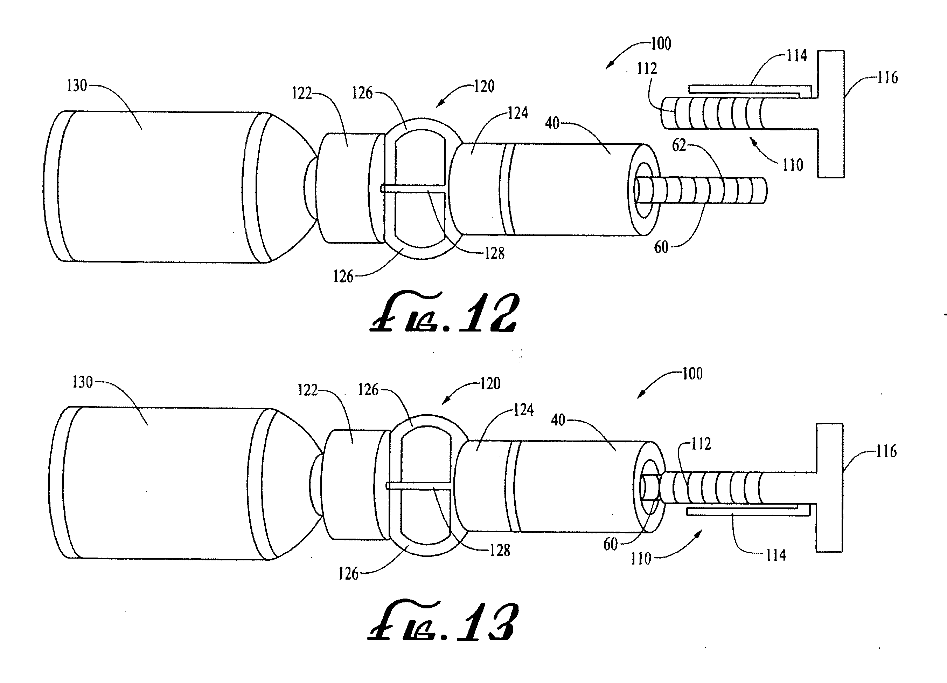

FIG. 12 illustrates another portion of an embodiment of the system 100 in accordance with an embodiment of the present invention. In the portion of the system 100 illustrated in the embodiment of FIG. 12, the system 100 includes the infusion medium container 130, the transfer guard 120, the reservoir 40, the plunger shaft 60, and the handle 110. In the embodiment of the system 100 illustrated in FIG. 12, the plunger shaft mating portion 62 of the plunger shaft 60 includes a threaded partial nut, and the handle mating portion 112 of the handle 110 includes a threaded interface. The handle mating portion 112 of the handle 110 is configured to be mated with the plunger shaft mating portion 62 of the plunger shaft. In various embodiments, the handle mating portion 112 is mated with the plunger shaft mating portion 62 by placing the handle mating portion 112 on the plunger shaft mating portion 62.

Placing the handle mating portion 112 on the plunger shaft mating portion 62 is only an example of a method of mating the handle 110 with the plunger shaft 60, and various other embodiments of the present invention are not limited to such a mating method. For example, various other embodiments may provide for the handle mating portion 112 and the plunger shaft mating portion 62 to be keyed, and for the handle mating portion 112 and the plunger shaft mating portion 62 to be mated by joining the keyed portions of each together. Also, various other embodiments may provide for the handle mating portion 112 to include a protrusion and for the plunger shaft mating portion 62 to include a corresponding groove so that the handle mating portion 112 is able to be mated with the plunger shaft mating portion 62 by placing the protrusion of the handle mating portion 112 in the groove of the plunger shaft mating portion 62. Various other mating methods are also possible.

In various embodiments, the handle 110 includes the gripping arm 114 for gripping the plunger shaft 60 when the handle mating portion 112 of the handle 110 is mated with the plunger shaft mating portion 62 of the plunger shaft 60. Also, in various embodiments, the handle 110 further includes a bar 116 connected to the handle mating portion 112, where the bar 116 is able to be gripped by the hand of a user to pull or push the handle 110 when the handle mating portion 112 is mated with the plunger shaft mating portion 62.

FIG. 30 illustrates another embodiment of the handle 110 in accordance with an embodiment of the present invention. The handle 110 illustrated in FIG. 30 includes the bar 116, the handle mating portion 112, and the gripping arm 114. The gripping arm 114 of the embodiment of the handle 110 in FIG. 30 is a skirt or sleeve that surrounds the handle mating portion 112.

FIG. 13 illustrates a portion of an embodiment of the system 100 in accordance with the embodiment shown in FIG. 12, where the handle mating portion 112 of the handle 110 has been mated with the plunger shaft mating portion 62 of the plunger shaft 60. As is illustrated in FIG. 13, in various embodiments, the handle mating portion 112 of the handle 110 is threaded and the plunger shaft mating portion 62 of the plunger shaft 60 is threaded, such that the handle mating portion 112 is able to mate with the plunger shaft mating portion 62 when the handle mating portion 112 is placed on the plunger shaft mating portion 62. In some embodiments, when the handle mating portion 112 is mated with the plunger shaft mating portion 62, the gripping arm 114 of the handle 110 is able to surround a perimeter of the plunger shaft 60 by 180° or more around the perimeter. In other embodiments, when the handle mating portion 112 is mated with the plunger shaft mating portion 62, the gripping arm 114 of the handle 110 is able to surround a perimeter of the plunger shaft 60 by only less than 180° around the perimeter. In some embodiments, when the handle mating portion 112 is mated with the plunger shaft mating portion 62, the gripping arm 114 of the handle 110 is able to surround a perimeter of the plunger shaft 60 by 360° around the perimeter.

In various embodiments, the handle 110 may be rotated when the handle mating portion 112 is mated with the plunger shaft mating portion 62 such that the handle 110 is positioned in a location where at least a portion of the plunger shaft 60 is located between the gripping arm 114 and at least a portion of the handle mating portion 112. Also, in various embodiments, the gripping arm 114 of the handle 110 may be biased so as to grip the plunger shaft 60 when the plunger shaft 60 is located between at least a portion of the gripping arm 114 and at least a portion of the handle mating portion 112. By gripping the plunger shaft 60 between at least a portion of the gripping arm 114 and at least a portion of the handle mating portion 112, the handle 110 may be prevented from disconnecting from the plunger shaft 60 until the handle 110 is rotated to a position where the plunger shaft 60 is not between the gripping arm 114 and the handle mating portion 112.

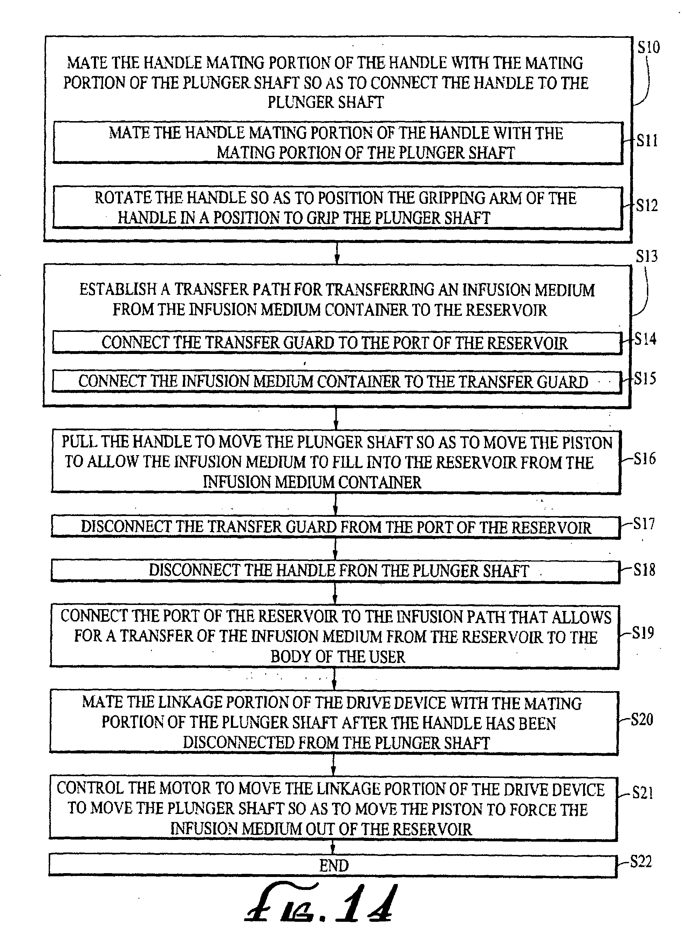

FIG. 14 illustrates a flow chart of a method of using an embodiment of the system 100 in accordance with an embodiment of the present invention. In describing the embodiment of the method illustrated by the flow chart in FIG. 14, reference will be made to various elements of embodiments of the system 100 illustrated in FIGs. 7-13. In S10, the handle mating portion 112 of the handle 110 is mated with the plunger shaft mating portion 62 of the plunger shaft 60, so as to connect the handle 110 to the plunger shaft 60. In various embodiments, the step S10 includes the step S11 of mating the handle mating portion 112 of the handle 110 with the plunger shaft mating portion 62 of the plunger shaft 60, and the step S12 of rotating the handle 110 so as to position the gripping arm 114 of the handle 110 in a position to grip the plunger shaft 60. The method then continues to S13.

In S13, a transfer path for transferring an infusion medium from the infusion medium container 130 to the reservoir 40 is established. In various embodiments, the step S13 includes the step S14 of connecting the transfer guard 120 to the port 41 of the reservoir 40, and the step S15 of connecting the infusion medium container 130 to the transfer guard 120. In some embodiments, the steps S10 and S13 are reversed such that a transfer path is established between the infusion medium container 130 and the reservoir 40 before the handle mating portion 112 is mated with the plunger shaft mating portion 62. The method then continues to S16.

In S16, the handle 110 is pulled to move the plunger shaft 60 so as to move the piston 70 to allow the infusion medium to flow or fill into the reservoir 40 from the infusion medium container 130, and the method continues to S17. In S17, the transfer guard 120 is disconnected from the port 41 of the reservoir 40, and the method continues to S18. In S18, the handle 110 is disconnected from the plunger shaft 60. The method then continues to S19.

In S19, the port 41 of the reservoir 40 is connected to the infusion path 50 that allows for a transfer of the infusion medium from the reservoir 40 to the body of a user, and the method continues to S20. In S20, the drive device linkage portion 82 of the drive device 80 is mated with the plunger shaft mating portion 62 of the plunger shaft 60 after the handle 110 has been disconnected from the plunger shaft 60. The method then continues to S21. In S21, the motor 84 is controlled to move the drive device linkage portion 82 of the drive device 80 to move the plunger shaft 60 so as to move the piston 70 to force the infusion medium out of the reservoir 40. The method then ends in S22.

In accordance with the embodiment of the method of using the system 100 illustrated in FIG. 14, the reservoir 40 is able to be filled with an infusion medium from the infusion medium container 130. In various embodiments, the reservoir 40 is housed in the reservoir retaining portion 24 of the disposable housing 20 (refer to FIGs. 6A and 6B), and the reservoir retaining portion 24 has an opening such that the reservoir 40 is able to be filled with an infusion medium from the infusion medium container 130 while the reservoir 40 is located within the reservoir retaining portion 24 of the disposable housing 20.

In various embodiments, the reservoir 40 is able to be refilled with an infusion medium using the method illustrated in FIG. 14. Thus, in various embodiments, the system 100 allows for filling and/or refilling of the reservoir 40. In some embodiments, the reservoir 40 may be partially filled with the infusion medium, while in other embodiments, the reservoir 40 may be completely filled with the infusion medium. Also, in some embodiments, the reservoir 40 may have measurement marks printed on a surface of the reservoir 40 so that the reservoir 40 can be filled with a measured amount of an infusion medium. Moreover, in various embodiments, the handle 110 is able to be pushed when the handle mating portion 112 is mated with the plunger shaft mating portion 62 so as to advance the piston 70 within the reservoir 40.

## Claims

1. A system for transferring an infusion medium, the system comprising:
a reservoir (40) for holding an infusion medium;
a piston (70) disposed at least partially within the reservoir (40), the piston being moveable to allow the infusion medium to fill into the reservoir (40)and to force the infusion medium out of the reservoir (40);
a plunger shaft (60) connected to the piston (70), the plunger shaft having a mating portion (62) for mating with a linkage portion (82) of a drive device (80), the drive device allowing for driving the plunger shaft (60) so as to move the piston (70) to force the infusion medium out of the reservoir when the linkage portion (82) of the drive device is mated with the mating portion (62) of the plunger shaft (60); and
a handle (110), the handle having a handle mating portion (112) for mating with the mating portion (62) of the plunger shaft (60), the handle capable of being used by a user to move the plunger shaft (60) so as to move the piston (70) to allow the infusion medium to fill into the reservoir (40) when the handle mating portion (112) of the handle (110) is mated with the mating portion (62) of the plunger shaft (60).

2. The system of claim 1,
wherein the mating portion (62) of the plunger shaft (60) is threaded.

3. The system of claim 1,
wherein the mating portion (62) of the plunger shaft (60) comprises a partial nut;
and
wherein the handle mating portion (112) of the handle (110) comprises a threaded interface.

4. The system of claim 1,
wherein the handle (110) has a gripping arm (114) for gripping the plunger shaft (60) when the handle mating portion (112) of the handle (110) is mated with the mating portion (62) of the plunger shaft (60).

5. The system of claim 1, further comprising:
a transfer guard (120) connectable to the reservoir (40) for providing a path to allow the infusion medium to be transferred from an infusion medium container (130) to the reservoir (40).

6. The system of claim 1, further comprising:
a base (21) adapted to be secured to a particular user;
wherein the reservoir (40) is connected to the base (21) or is integrated into the base (21).

7. The system of claim 1, further comprising:
the drive device (80) having the linkage portion (82);
wherein the drive device further comprises a motor (84) for moving the linkage portion (82); and
wherein the motor (84) is able to move the linkage portion (82) of the drive device (80) so as to drive the plunger shaft (60) when the linkage portion (82) of the drive device (80) is mated with the mating portion (62) of the plunger shaft (60).

8. The system of claim 1,
wherein the reservoir (40) has a degassing portion (42) that comprises a hydrophobic material for allowing gases to escape from the reservoir (40) while keeping the infusion medium within the reservoir (40).

9. The system of claim 1,
wherein the piston (70) has a degassing portion (72) that comprises a hydrophobic material for allowing gases to escape from the reservoir (40) while keeping the infusion medium within the reservoir (40).

10. A method for using a system for transferring an infusion medium, the system comprising a reservoir (40), a piston (70) disposed at least partially within the reservoir (40), a plunger shaft (60) connected to the piston (70), and a handle (110), the plunger shaft (60) having a mating portion (62) for mating with a linkage portion (82) of a drive device (80), the handle (110) having a handle mating portion (112) for mating with the mating portion (62) of the plunger shaft (60), the method comprising:
mating the handle mating portion (112) of the handle with the mating portion (62) of the plunger shaft (60) so as to connect the handle (110) to the plunger shaft (60);
establishing a transfer path for transferring an infusion medium from an infusion medium container (130) to the reservoir (40); and
pulling the handle (110) to move the plunger shaft (60) so as to move the piston (70) to allow the infusion medium to fill into the reservoir (40) from the infusion medium container (130).

11. The method of claim 10, further comprising:
disconnecting the handle (110) from the plunger shaft (60).

12. The method of claim 10, further comprising:
mating the linkage portion (82) of the drive device (80) with the mating portion (62) of the plunger shaft (60) after the handle (110) has been disconnected from the plunger shaft (60).

13. The method of claim 10,
wherein the handle comprises a gripping arm (114); and
wherein the step of mating the handle mating portion (112) of the handle with the mating portion (62) of the plunger shaft (60), comprises:
mating the handle mating portion (112) of the handle with the mating portion (62) of the plunger shaft (60); and
rotating the handle (110) so as to position the gripping arm (114) of the handle in a position to grip the plunger shaft (60).

14. The method of claim 10, wherein the step of establishing the transfer path, comprises:
connecting a transfer guard (120) to a port of the reservoir (40); and
connecting the infusion medium container (130) to the transfer guard (120).

15. The method of claim 14, further comprising:
disconnecting the transfer guard (120) from the port of the reservoir (40); and
connecting the port of the reservoir (40) to an infusion path (50) that allows for a transfer of the infusion medium from the reservoir to a body of a user.

## Patentansprüche

1. System zum Transferieren eines Infusionsmediums, mit:
einem Reservoir (40) zur Aufnahme eines Infusionsmediums;
einem Kolben (70), der zumindest teilweise innerhalb des Reservoirs (40) angeordnet ist, wobei der Kolben bewegbar ist, um dem Infusionsmedium zu erlauben, das Reservoir (40) zu füllen und das Infusionsmedium aus dem Reservoir (40) zu drängen;
einem Plungerschaft (60), der mit dem Kolben (70) verbunden ist, wobei der Plungerschaft einen Eingriffsabschnitt (62) zum Eingriff mit einem Verbindungsabschnitt (82) einer Antriebsvorrichtung (80) aufweist, wobei die Antriebsvorrichtung erlaubt, den Plungerschaft (60) anzutreiben, um den Kolben (70) zu bewegen, um das Infusionsmedium aus dem Reservoir zu drängen, wenn der Verbindungsabschnitt (82) der Antriebsvorrichtung im Eingriff mit dem Eingriffsabschnitt (62) des Plungerschafts (60) ist; und
einem Griff (110), wobei der Griff einen Griffeingriffsabschnitt (112) zum Eingriff mit dem Eingriffsabschnitt (62) des Plungerschafts (60) aufweist, wobei der Griff geeignet ist, von einem Benutzer verwendet zu werden, um den Plungerschaft (60) zu bewegen, um den Kolben (70) zu bewegen, um dem Infusionsmedium zu erlauben, das Reservoir (40) zu füllen, wenn der Griffeingriffsabschnitt (112) des Griffs (110) im Eingriff mit dem Eingriffsabschnitt (62) des Plungerschafts (60) ist.

2. System nach Anspruch 1,
bei dem der Eingriffsabschnitt (62) des Plungerschafts (60) mit einem Gewinde versehen ist.

3. System nach Anspruch 1, bei dem
der Eingriffsabschnitt (62) des Plungerschafts (60) eine partielle Mutter umfasst; und
der Griffeingriffsabschnitt (112) des Griffs (110) eine Schnittstelle aufweist, die mit einem Gewinde versehen ist.

4. System nach Anspruch 1, bei dem der Griff (110) einen Greifarm (114) zum Greifen des Plungerschafts (60) aufweist, wenn der Griffeingriffsabschnitt (112) des Griffs (110) im Eingriff mit dem Eingriffsabschnitt (62) des Plungerschafts (60) ist.

5. System nach Anspruch 1, weiterhin mit:
einem Transferschutz (120), der mit dem Reservoir (40) verbindbar ist, um einen Pfad bereitzustellen, um dem Infusionsmedium zu erlauben, aus einem Infusionsmediumsbehälter (130) in das Reservoir (40) transferiert zu werden.

6. System nach Anspruch 1, weiterhin mit:
einer Basis (21), die ausgebildet ist, an einem bestimmten Benutzer befestigt zu werden;
wobei das Reservoir (40) mit der Basis (21) verbunden ist oder in die Basis (21) integriert ist.

7. System nach Anspruch 1:
wobei die Antriebsvorrichtung (80) den Verbindungsabschnitt (82) aufweist;
wobei die Antriebsvorrichtung weiterhin einen Motor (84) zum Bewegen des Verbindungsabschnitts (82) aufweist; und
wobei der Motor (84) geeignet ist den Verbindungsabschnitt (82) der Antriebsvorrichtung (80) zu bewegen, um den Plungerschaft (60) anzutreiben, wenn der Verbindungsabschnitt (82) der Antriebsvorrichtung (80) mit dem Eingriffsabschnitt (62) des Plungerschafts (60) im Eingriff ist.

8. System nach Anspruch 1,
wobei das Reservoir (40) einen Entlüftungsabschnitt (42) aufweist, der ein hydrophobes Material umfasst, um Gasen zu erlauben, aus dem Reservoir (40) zu entweichen, während das Infusionsmedium in dem Reservoir (40) gehalten wird.

9. System nach Anspruch 1,
wobei der Kolben (70) einen Entlüftungsabschrutt (72) aufweist, der ein hydrophobes Material umfasst, um Gasen zu erlauben, aus dem Reservoir (40) zu entweichen, während das Infusionsmedium in dem Reservoir (40) gehalten wird.

10. Verfahren zur Verwendung eines Systems zum Übertragen eines Infusionsmediums, mit einem Reservoir (40), einem Kolben (70), der zumindest teilweise innerhalb des Reservoirs (40) angeordnet ist, einem Plungerschaft (60), der mit dem Kolben (70) verbunden ist, und mit einem Griff (110), wobei der Plungerschaft (60) einen Eingriffsabschnitt (62) zum Eingriff mit einem Verbindungsabschnitt (82) einer Antriebsvorrichtung (80) aufweist, der Griff (110) einen Griffeingriffsabschnitt (112) zum Eingriff mit dem Eingriffsabschnitt (62) des Plungerschafts (60) aufweist, wobei das Verfahren umfasst:
In-Eingriff-Bringen des Griffeingriffsabschnitts (112) des Griffs mit dem Eingriffsabschnitt (62) des Plungerschafts (60), um den Griff (110) mit dem Plungerschaft (60) zu verbinden;
Schaffen eines Transferpfades zum Transferieren eines Infusionsmediums aus einem Infusionsmediumsbehälter (130) in das Reservoir (40); und
Ziehen des Griffs (110), um den Plungerschaft (60) zu bewegen, um den Kolben (70) zu bewegen, um dem Infusionsmedium zu erlauben, das Reservoir (40) aus dem Infusionsmediumsbehälter (130) zu füllen.

11. Verfahren nach Anspruch 10, weiterhin mit:
Trennen des Griffs (110) von dem Plungerschaft (60).

12. Verfahren nach Anspruch 10, weiterhin mit:
In-Eingriff-Bringen des Verbindungsabschnitts (82) der Antriebsvorrichtung (80) mit dem Eingriffsabschnitt (62) des Plungerschafts (60) nachdem der Griff (110) von dem Plungerschaft (60) getrennt wurde.

13. Verfahren nach Anspruch 10,
wobei der Griff einen Greifarm (114) umfasst; und
wobei der Schritt des In-Eingriff-Bringens des Griffeingriffsabschnitts (112) des Griffs mit dem Eingriffsabschnitt (62) des Plungerschafts (60) umfasst:
In-Eingriff-Bringen des Griffeingriffsabschnitts (112) des Griffs mit dem Eingriffsabschnitt (62) des Plungerschafts (60); und
Drehen des Griffs (110), um den Greifarm (114) des Griffs in einer Stellung zu positionieren, um den Plungerschaft (60) zu greifen.

14. Verfahren nach Anspruch 10, bei dem der Schritt des Schaffens eines Transferpfades umfasst:
Verbinden eines Transferschutzes (120) mit einem Anschluss des Reservoirs (40); und
Verbinden des Infusionsmediumsbehälters (130) mit dem Transferschutz (120).

15. Verfahren nach Anspruch 14, weiterhin mit:
Trennen des Transferschutzes (21) von dem Anschluss des Reservoirs (40);
und
Verbinden des Anschlusses des Reservoirs (40) mit einem Infusionspfad (50), der einen Transfer des Infusionsmediums von dem Reservoir in einen Körper eines Benutzers erlaubt.

## Revendications

1. Système pour transférer un milieu de perfusion, le système comprenant :
un réservoir (40) pour contenir un milieu de perfusion ;
un piston (70) disposé au moins partiellement à l'intérieur du réservoir (40), le piston étant mobile pour permettre au milieu de perfusion de remplir le réservoir (40) et pour forcer le milieu de perfusion hors du réservoir (40) ;
une tige de piston plongeur (60) reliée au piston (70), la tige de piston plongeur comportant une partie d'accouplement (62) pour s'accoupler avec une partie de liaison (82) d'un dispositif d'entraînement (80), le dispositif d'entraînement permettant d'entraîner la tige de piston plongeur (60) afin de déplacer le piston (70) pour forcer le milieu de perfusion hors du réservoir lorsque la partie de liaison (82) du dispositif d'entraînement est accouplée avec la partie d'accouplement (62) de la tige de piston plongeur (60) ; et
une poignée (110), la poignée comportant une partie d'accouplement de poignée (112) pour s'accoupler avec la partie d'accouplement (62) de la tige de piston plongeur (60), la poignée étant capable d'être utilisée par un utilisateur pour déplacer la tige de piston plongeur (60) afin de déplacer le piston (70) pour permettre au milieu de perfusion de remplir le réservoir (40) lorsque la partie d'accouplement de poignée (112) de la poignée (110) est accouplée avec la partie d'accouplement (62) de la tige de piston plongeur (60).

2. Système selon la revendication 1, dans lequel
la partie d'accouplement (62) de la tige de piston plongeur (60) est filetée.

3. Système selon la revendication 1,
dans lequel la partie d'accouplement (62) de la tige de piston plongeur (60) comprend un écrou partiel ; et
dans lequel la partie d'accouplement de poignée (112) de la poignée (110) comprend une interface filetée.

4. Système selon la revendication 1, dans lequel
la poignée (110) comporte un bras de préhension (114) pour saisir la tige de piston plongeur (60) lorsque la partie d'accouplement de poignée (112) de la poignée (110) est accouplée avec la partie d'accouplement (62) de la tige de piston plongeur (60).

5. Système selon la revendication 1, comprenant en outre :
une gaine de transfert (120) raccordable avec le réservoir (40) pour fournir un trajet pour permettre au milieu de perfusion d'être transféré d'un contenant de milieu de perfusion (130) au réservoir (40).

6. Système selon la revendication 1, comprenant en outre :
une base (21) adaptée pour être attachée à un utilisateur particulier ;
dans lequel le réservoir (40) est relié à la base (21) ou est intégré dans la base (21).

7. Système selon la revendication 1, comprenant en outre :
le dispositif d'entraînement (80) comportant la partie de liaison (82) ;
dans lequel le dispositif d'entraînement comprend en outre un moteur (84) pour déplacer la partie de liaison (82) ; et
dans lequel le moteur (84) est capable de déplacer la partie de liaison (82) du dispositif d'entraînement (80) afin d'entraîner la tige de piston plongeur (60) lorsque la partie de liaison (82) du dispositif d'entraînement (80) est accouplée avec la partie d'accouplement (62) de la tige de piston plongeur (60).

8. Système selon la revendication 1, dans lequel le réservoir (40) comporte une partie de dégazage (42) qui comprend un matériau hydrophobe pour permettre à des gaz de s'échapper du réservoir (40) tout en maintenant le milieu de perfusion à l'intérieur du réservoir (40).

9. Système selon la revendication 1, dans lequel
le piston (70) comporte une partie de dégazage (72) qui comprend un matériau hydrophobe pour permettre à des gaz de s'échapper du réservoir (40) tout en maintenant le milieu de perfusion à l'intérieur du réservoir (40).

10. Procédé pour utiliser un système pour transférer un milieu de perfusion, le système comprenant un réservoir (40), un piston (70) disposé au moins partiellement à l'intérieur du réservoir (40), une tige de piston plongeur (60) reliée au piston (70), et une poignée (110), la tige de piston plongeur (60) comportant une partie d'accouplement (62) pour s'accoupler avec une partie de liaison (82) d'un dispositif d'entraînement (80), la poignée (110) comportant une partie d'accouplement de poignée (112) pour s'accoupler avec la partie d'accouplement (62) de la tige de piston plongeur (60), le procédé comprenant :
l'accouplement de la partie d'accouplement de poignée (112) de la poignée avec la partie d'accouplement (62) de la tige de piston plongeur (60) afin de relier la poignée (110) à la tige de piston plongeur (60) ;
l'établissement d'un trajet de transfert pour transférer un milieu de perfusion d'un contenant de milieu de perfusion (130) au réservoir (40) ; et
la traction de la poignée (110) pour déplacer la tige de piston plongeur (60) afin de déplacer le piston (70) pour permettre au milieu de perfusion de remplir le réservoir (40) à partir du contenant de milieu de perfusion (130).

11. Procédé selon la revendication 10, comprenant en outre :
la déconnexion de la poignée (110) de la tige de piston plongeur (60).

12. Procédé selon la revendication 10, comprenant en outre :
l'accouplement de la partie de liaison (82) du dispositif d'entraînement (80) avec la partie d'accouplement (62) de la tige de piston plongeur (60) après que la poignée (110) a été déconnectée de la tige de piston plongeur (60).

13. Procédé selon la revendication 10,
dans lequel la poignée comprend un bras de préhension (114) ; et
dans lequel l'étape d'accouplement de la partie d'accouplement de poignée (112) de la poignée avec la partie d'accouplement (62) de la tige de piston plongeur (60) comprend :
- l'accouplement de la partie d'accouplement de poignée (112) de la poignée avec la partie d'accouplement (62) de la tige de piston plongeur (60) ; et
- la rotation de la poignée (110) afin de positionner le bras de préhension (114) de la poignée dans une position pour saisir la tige de piston plongeur (60).

14. Procédé selon la revendication 10, dans lequel l'étape d'établissement du trajet de transfert, comprend :
la connexion d'une gaine de transfert (120) à un orifice du réservoir (40) ; et
la connexion du contenant de milieu de perfusion (130) à la gaine de transfert (120).

15. Procédé selon la revendication 14, comprenant en outre :
la déconnexion de la gaine de transfert (120) de l'orifice du réservoir (40) ; et
la connexion de l'orifice du réservoir (40) à un trajet de perfusion (50) qui permet un transfert du milieu de perfusion du réservoir au corps d'un utilisateur.
